# EUROPEAN PATENT APPLICATION

(11) **EP 1 236 727 A1**
(43) Date of publication of application: **04.09.2002**
(21) Application number: 02004472.3
(22) Date of filing: 27.02.2002
(51) Int. Cl.: C07D 487/08, A01N 43/90

(54) **Azabicyclo compounds as pesticides**

(30) Priority: 01.03.2001 EP 01105017
(71) Applicant: Bayer CropScience GmbH, 65929 Frankfurt/Main (DE)
(72) Inventor: Lindell, Stephen Dr., 65779 Kelkheim-Fishbach (DE); Dickhaut, Joachim Dr., 69121 Heidelberg (DE); Jakobi, Harald Dr., 60598 Frankfurt (DE); Tiebes, Jörg Dr., 60431 Frankfurt (DE); Jans, Daniela Dr., 61348 Bad Homburg V.D.H. (DE); Thönessen, Maria-Theresia, 55262 Heidesheim (DE); Waibel, Jutta Maria, 60529 Frankfurt (DE)

(57) **Abstract**

Azabicyclo compounds of the formula (I) and (II), 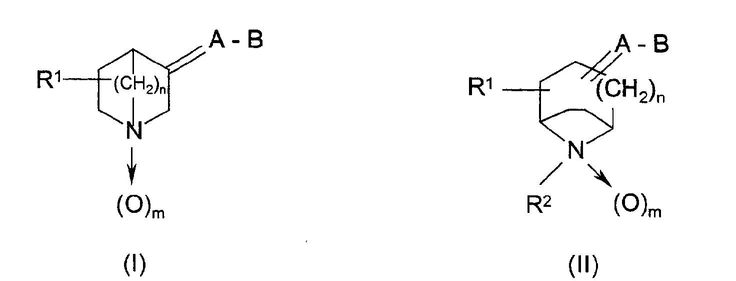 wherein B is a thiadiazolyl residue and the other symbols and indices have the meanings given in the specification, are useful for controlling animal pests.

## Description

The invention relates to the field of pest control, especially the control of animal pests like arthropods, e.g. insects and arachnids, ecto- and endoparasites, helminths, nematodes and mollusks.

Carbamates of quinuclidinone 3-oximes are known as pesticides from FR-A 2 086 292.

However, since modem pesticides must meet a wide range of demands, for example regarding level, duration and spectrum of action, use spectrum, toxicity, combination with other active substances, combination with formulation auxiliaries or synthesis, and since the occurrence of resistances is possible, the development of such substances can never be regarded as concluded, and there is constantly a high demand for novel pesticides which are advantageous over the known ones, at least as far as some aspects are concerned.

It was an object of the present invention to provide compounds which widen the spectrum of existing pesticides in various aspects.

It has now surprisingly been found that certain azabicyclo compounds are useful for controlling animal pests.

Therefore, in one aspect of the invention there are provided azabicyclo compounds of the formulae (I) and (II) or agrochemically acceptable salts thereof, 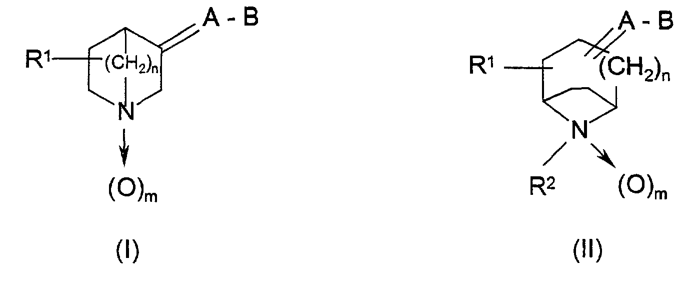 wherein
- m: is zero or one;
- n: is one or two
- R¹: is hydrogen, a straight or branched lower alkyl group having from 1 to 6 carbon atoms, (C₃-C₆)-cycloalkyl, hydroxy, a straight or branched lower alkoxy group having from 1 to 4 carbon atoms, an acyloxy group, wherein the acyl moiety has from 2 to 5 carbon atoms;
- R²: is hydrogen or a straight or branched lower alkyl group having from 1 to 4 carbon atoms, which is optionally substituted by one or more fluorine atoms;
- A: is N-O-CHR³, or CR⁴;
- B: is 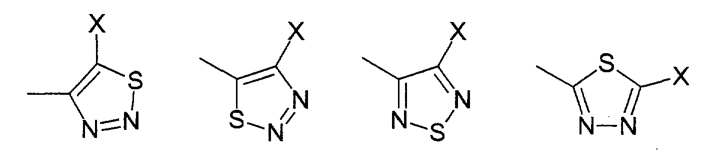 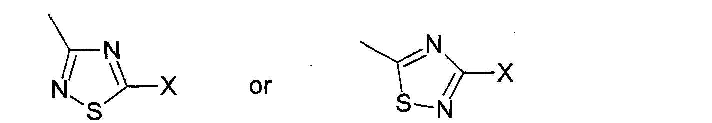
- X: is H or a substituent;
- R³: is H, (C₁-C₄)-alkyl, (C₂-C₄)-alkenyl, (C₂-C₄)-alkynyl, (C₁-C₆)-cycloalkyl;
- R⁴: is H, halogen, CN, (C₁-C₄)-alkyl, (C₂-C₄)-alkenyl, (C₂-C₄)-alkynyl, (C₁-C₆)-cycloalkyl, -O-(C₁-C₄)-alkyl, -O-(C₂-C₄)-alkenyl, -O-(C₂-C₄)-alkynyl, -O-(C₁-C₆)-cycloalkyl, -S-(C₁-C₄)-alkyl, -S-(C₂-C₄)-alkenyl, -S-(C₂-C₄)-alkynyl, -S-(C₁-C₆)-cycloalkyl, -NH-(C₁-C₄)-alkyl, -NH-(C₂-C₄)-alkenyl, -NH-(C₂-C₄)-alkynyl, -NH-(C₁-C₆)-cycloalkyl, N((C₁-C₄)-alkyl)₂, N((C₁-C₆)-cycloalkyl)₂, morpholinyl, piperidyl, pyrrolidinyl;

In the compounds depicted by general Formulae (I), and (II) the various substituents are further described as follows. Illustrative examples of straight or branched lower alkyl having from 1 to 4 carbon atoms or from 1 to 6 carbon atoms include methyl, trifluoromethyl, ethyl, 2,2,2-trifluoroethyl, n-propyl, isopropyl, n-butyl, tert.-butyl, n-pentyl and n-hexyl.

Illustrative examples of lower alkoxy groups having from 1 to 4 carbon atoms are methoxy, trifluoromethoxy, ethoxy and n-propoxy.

Illustrative examples of a saturated straight or branched hydrocarbon chain having from 1 to 8 carbon atoms include n-octyl, n-heptyl, and all the illustrative examples of straight or branched lower alkyl groups having from 1 to 6 carbon atoms set forth above.
Illustrative examples of straight or branched unsaturated hydrocarbon chains which contain from 1 to 3 unsaturations which are double or triple bonds are ethenyl, 2,4-pentadienyl, 1,4-pentadienyl, 2,4-pentadiynyl, 1,4-pentadiynyl, (E&Z)-2-penten-4-ynyl, 2-pentyn-4-enyl, 2-propenyl, 3-butenyl, 1-methyl-2-propenyl, 2-pentenyl, 2-ethyl-3-butenyl, 4-hexenyl, ethynyl, 2-propynyl, 1-methyl-2-propynyl, 1-ethyl-2-propynyl, 1-methyl-3-butynyl, 3-butynyl, or 4-pentynyl. Illustrative examples of cycloalkyl groups having from 3 to 6 carbon atoms are cyclopropyl, cyclobutyl and cyclohexyl.

Agrochemically acceptable acid addition salts of the compounds of Formulae (I) and (II) are illustratively hydrochloric, hydrobromic, sulfuric, phosphoric, acetic, benzoic, citric, malonic, salicylic, malic, fumaric, oxalic, succinic, tartaric, lactic, gluconic, ascorbic, maleic, asparatic, benzenesulfonic, methane- and ethanesulfonic, hydroxymethane- und hydroxyethanesulfonic.

Preferred compounds of the present invention are those of Formulae (I). The most preferred compounds of this invention are those of Formula (I) wherein n is one, i.e., compounds of the following Formula (III): 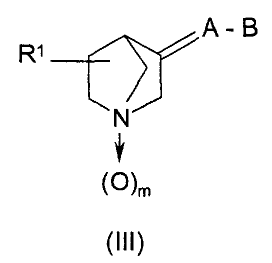 where A, B, m and R¹ have the same meanings as set forth above for Formula (I), and (II). Compounds of Formula (III) wherein R¹ is hydrogen are more preferred.

Compounds of Formula (I) wherein n is two may be depicted as set forth below in Formula (IV): 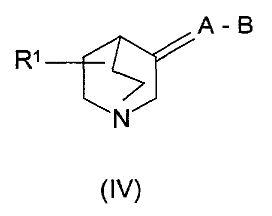 wherein A, B and R¹ have the meanings defined above.
- A: is preferably =N-O-CH₂-.
- B: is preferably a substituted thiadiazole group, more preferably substituted 1,2,3-thiadiazole.
- R³: is preferably H or CH₃, more preferably H.
- R⁴: is preferably H, halogen or CH₃, more preferably H.

In the compounds of Formulae (II) the A-B group is attached to the 2-, 3- or 4-position (Formula II) of the ring.

Compounds of the present invention may exist in either of two Z and E isomeric forms. The present invention includes both forms of the compounds as well as mixtures of the Z and E forms. Illustratively Formulae (V) and (VI) depict the Z and E forms of the compounds of Formula (I). Moreover, in those compounds in which there is a double bond in a carbon chain, both the Z and E forms of the olefins are included in the present invention. 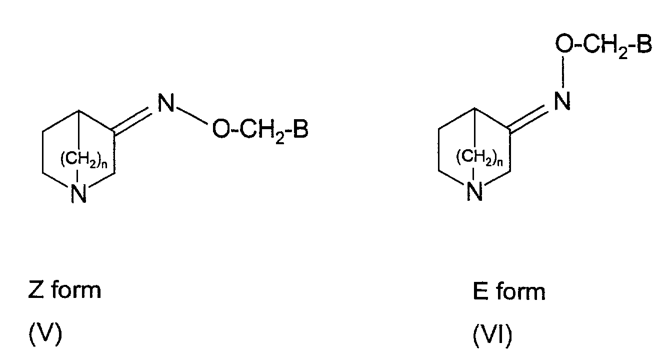
- X: is preferably R⁵, R⁶, -C(W)R⁵, -C(=NOR⁵)R⁵,-C(=NNR⁵₂)R⁵, -C(=W)OR⁵, -C(=W)NR⁵₂, -OC(=W)R⁵, -OC(=W)OR⁵, -NR⁵C(=W)R⁵, -N[C(=W)R⁵]₂, -NR⁵C(=W)OR⁵, -C(=W)NR⁵-NR⁵₂, -C(=W)NR⁵-NR⁵[C(=W)R⁵], -NR⁵-C(=W)NR⁵₂, -NR⁵-NR⁵C(=W)R⁵, -NR⁵-N[C(=W)R⁵]₂, -N[(C=W)R⁵]-NR⁵₂, -NR⁵-N[(C=W)WR⁵], -NR⁵[(C=W)NR⁵₂], -NR⁵(C=NR⁵)R⁵, -NR⁵(C=NR⁵)NR⁵₂, -O-NR⁵₂, -O-NR⁵(C=W)R⁵, -SO₂NR⁵₂, -NR⁵SO₂R⁵, -SO₂OR⁵, -OSO₂R⁵, -OR⁵, -NR⁵₂, -SR⁵, -SiR⁵₃, -PR⁵₂, -P(=W)R⁵₂, -SOR⁵, -SO₂R⁵, -PW₂R⁵₂, -PW₃R⁵₂;
- W: is O or S;
- R⁵: are identical or different and are (C₁-C₆)-alkyl, (C₂-C₆)-alkenyl, (C₂-C₆)-alkynyl, (C₃-C₈)-cycloalkyl, (C₄-C₈)-cycloalkenyl, (C₃-C₈)-cycloalkyl-(C₁-C₄)-alkyl, (C₄-C₈)-cycloalkenyl-(C₁-C₄)-alkyl, (C₃-C₈)-cycloalkyl-(C₂-C₄)-alkenyl, (C₄-C₈)-cycloalkenyl-(C₂-C₄)-alkenyl, (C₁-C₆)-alkyl-(C₃-C₈)-cycloalkyl, (C₂-C₆)-alkenyl-(C₃-C₈)-cycloalkyl, (C₂-C₆)-alkynyl-(C₃-C₈)-cycloalkyl, (C₁-C₆)-alkyl-(C₄-C₈)-cycloalkenyl, (C₂-C₆)-alkenyl-(C₄-C₈)-cycloalkenyl, aryl, heterocyclyl;
where the radicals mentioned are optionally substituted by one or more radicals R⁶;
- R⁶: is identical or different halogen, cyano, nitro, hydroxyl, thio, amino, formyl, (C₁-C₆)-alkoxy, (C₁-C₆)-alkanoyl, (C₃-C₆)-alkenyloxy, (C₃-C₆)-alkynyloxy, (C₁-C₆)-haloalkyloxy, (C₃-C₆)-haloalkenyloxy, (C₃-C₆)-haloalkynyloxy, (C₃-C₈)-cycloalkoxy, (C₄-C₈)-cycloalkenyloxy, (C₃-C₈)-halocycloalkoxy, (C₄-C₈)-halocycloalkenyloxy, (C₃-C₈)-cycloalkyl-(C₁-C₄)-alkoxy,
(C₄-C₈)-cycloalkenyl-(C₁-C₄)-alkoxy, (C₃-C₈)-cycloalkyl-(C₂-C₄)-alkenyloxy, (C₄-C₈)-cycloalkenyl-(C₁-C₄)-alkenyloxy, (C₁-C₆)-alkyl-(C₃-C₈)-cycloalkoxy, (C₂-C₆)-alkenyl-(C₃-C₈)-cycloalkoxy, (C₂-C₆)-alkynyl-(C₃-C₈)-cycloalkoxy, (C₁-C₆)-alkyl-(C₄-C₈)-cycloalkenyloxy, (C₂-C₆)-alkenyl-(C₄-C₈)-cycloalkenyloxy, (C₁-C₄)-alkoxy-(C₁-C₆)-alkoxy, (C₁-C₄)-alkoxy-(C₃-C₆)-alkenyloxy, carbamoyl, (C₁-C₆)-mono- or dialkylcarbamoyl, (C₁-C₆)-mono- or dihaloalkylcarbamoyl, (C₃-C₈)-mono- or dicycloalkylcarbamoyl, (C₁-C₆)-alkoxycarbonyl, (C₃-C₈)-cycloalkoxycarbonyl, (C₁-C₆)-alkanoyloxy, (C₃-C₈)-cycloalkanoyloxy, (C₁-C₆)-haloalkoxycarbonyl, (C₁-C₆)-haloalkanoyloxy, (C₁-C₆)-alkanamido, (C₁-C₆)-haloalkanamido, (C₂-C₆)-alkenamido, (C₃-C₈)-cycloalkanamido, (C₃-C₈)-cycloalkyl-(C₁-C₄)-alkanamido, (C₁-C₆)-alkylthio, (C₃-C₆)-alkenylthio, (C₃-C₆)-alkynylthio, (C₁-C₆)-haloalkylthio, (C₃-C₆)-haloalkenylthio, (C₃-C₆)-haloalkynyithio, (C₃-C₈)-cycloalkylthio, (C₄-C₈)-cycloalkenylthio, (C₃-C₈)-halocycloalkylthio, (C₄-C₈)-halocycloalkenylthio, (C₃-C₈)-cycloalkyl-(C₁-C₄)-alkylthio, (C₄-C₈)-cycloalkenyl-(C₁-C₄)-alkylthio, (C₃-C₈)-cycloalkyl-(C₃-C₄)-alkenylthio, (C₄-C₈)-cycloalkenyl-(C₃-C₄)-alkenylthio, (C₁-C₆)-alkyl-(C₃-C₈)-cycloalkylthio, (C₂-C₆)-alkenyl-(C₃-C₈)-cycloalkylthio, (C₂-C₆)-alkynyl-(C₃-C₈)-cycloalkylthio, (C₁-C₆)-alkyl-(C₄-C₈)-cycloalkenylthio, (C₂-C₆)-alkenyl-(C₄-C₈)-cycloalkenylthio, (C₁-C₆)-alkylsulfinyl, (C₃-C₆)-alkenylsulfinyl, (C₃-C₆)-alkynylsulfinyl, (C₁-C₆)-haloalkylsulfinyl, (C₃-C₆)-haloalkenylsulfinyl, (C₃-C₆)-haloalkynylsulflnyl, (C₃-C₈)-cycloalkylsulfinyl, (C₄-C₈)-cycloalkenylsulfinyl, (C₃-C₈)-halocycloalkylsulfinyl, (C₄-C₈)-halocycloalkenylsulfinyl, (C₃-C₈)-cycloalkyl-(C₁-C₄)-alkylsulfinyl,
(C₄-C₈)-cycloalkenyl-(C₁-C₄)-alkylsulfinyl, (C₃-C₈)-cycloalkyl-(C₃-C₄)-alkenylsulfinyl, (C₄-C₈)-cycloalkenyl-(C₃-C₄)-alkenylsulfinyl, (C₁-C₆)-alkyl-(C₃-C₈)-cycloalkylsulfinyl, (C₂-C₆)-alkenyl-(C₃-C₈)-cycloalkylsulfinyl, (C₂-C₆)-alkynyl-(C₃-C₈)-cycloalkylsulfinyl, (C₁-C₆)-alkyl-(C₄-C₈)-cycloalkenylsulfinyl, (C₂-C₆)-alkenyl-(C₄-C₈)-cycloalkenylsulfinyl, (C₁-C₆)-alkylsulfonyl, (C₃-C₆)-alkenylsulfonyl, (C₃-C₆)-alkynylsulfonyl, (C₁-C₆)-haloalkylsulfonyl, (C₃-C₆)-haloalkenylsulfonyl, (C₃-C₆)-haloalkynylsulfonyl, (C₃-C₈)-cycloalkylsulfonyl, (C₄-C₈)-cycloalkenylsulfonyl, (C₃-C₈)-halocycloalkylsulfonyl, (C₄-C₈)-halocycloalkenylsulfonyl, (C₃-C₈)-cycloalkyl-(C₁-C₄)-alkylsulfonyl, (C₄-C₈)-cycloalkenyl-(C₁-C₄)-alkylsulfonyl, (C₃-C₈)-cycloalkyl-(C₃-C₄)-alkenylsulfonyl, (C₄-C₈)-cycloalkenyl-(C₃-C₄)-alkenylsulfonyl, (C₁-C₆)-alkyl-(C₃-C₈)-cycloalkylsulfonyl, (C₂-C₆)-alkenyl-(C₃-C₈)-cycloalkylsulfonyl, (C₂-C₆)-alkynyl-(C₃-C₈)-cycloalkylsulfonyl, (C₁-C₆)-alkyl-(C₄-C₈)-cycloalkenylsulfonyl, (C₂-C₆)-alkenyl-(C₄-C₈)-cycloalkenylsulfonyl, (C₁-C₆)-alkylamino, (C₃-C₆)-alkenylamino, (C₃-C₆)-alkynylamino, (C₁-C₆)-haloalkylamino, (C₃-C₆)-haloalkenylamino, (C₃-C₆)-haloalkynylamino, (C₃-C₈)-cycloalkylamino, (C₄-C₈)-cycloalkenylamino, (C₃-C₈)-halocycloalkylamino, (C₄-C₈)-halocycloalkenylamino, (C₃-C₈)-cycloalkyl-(C₁-C₄)-alkylamino, (C₄-C₈)-cycloalkenyl-(C₁-C₄)-alkylamino, (C₃-C₈)-cycloalkyl-(C₃-C₄)-alkenylamino, (C₄-C₈)-cycloalkenyl-(C₃-C₄)-alkenylamino, (C₁-C₆)-alkyl-(C₃-C₈)-cycloalkylamino, (C₂-C₆)-alkenyl-(C₃-C₈)-cycloalkylamino, (C₂-C₆)-alkynyl-(C₃-C₈)-cycloalkylamino, (C₁-C₆)-alkyl-(C₄-C₈)-cycloalkenylamino, (C₂-C₆)-alkenyl-(C₄-C₈)-cycloalkenylamino, (C₁-C₆)-trialkylsilyl, aryl, aryloxy, arylthio, arylamino, aryl-(C₁-C₄)-alkoxy, aryl-(C₃-C₄)-alkenyloxy, aryl-(C₁-C₄)-alkylthio, aryl-(C₂-C₄)-alkenylthio, aryl-(C₁-C₄)-alkylamino, aryl-(C₃-C₄)-alkenylamino, aryl-(C₁-C₆)-dialkylsilyl, diaryl-(C₁-C₆)-alkylsilyl, triarylsilyl and 5- or 6-membered heterocyclyl, the cyclic moiety of the fourteen last-mentioned radicals being optionally substituted by one or more radicals selected from the group consisting of halogen, cyano, nitro, amino, hydroxyl, thio, (C₁-C₄)-alkyl, (C₁-C₄)-haloalkyl, (C₃-C₈)-cycloalkyl, (C₁-C₄)-alkoxy, (C₁-C₄)-haloalkoxy, (C₁-C₄)-alkylthio, (C₁-C₄)-haloalkylthio, (C₁-C₄)-alkylamino, (C₁-C₄)-haloalkylamino, formyl and (C₁-C₄)-alkanoyl.

The term "halogen" embraces fluorine, chlorine, bromine and iodine. The expression "(C₁-C₄)-alkyl" is to be understood as meaning an unbranched or branched hydrocarbon radical having 1, 2, 3 or 4 carbon atoms, such as, for example, the methyl, ethyl, propyl, isopropyl, 1-butyl, 2-butyl, 2-methylpropyl or tert-butyl radical. Correspondingly, alkyl radicals having a larger range of carbon atoms are to be understood as meaning an unbranched or branched saturated hydrocarbon radical which contains a number of carbon atoms which corresponds to this stated range. Accordingly, the expression "(C₁-C₆)-alkyl" embraces the abovementioned alkyl radicals, and also, for example, the pentyl, 2-methylbutyl, 1,1-dimethylpropyl or hexyl radical. The expression "(C₁-C₁₀)-alkyl" is to be understood as meaning the abovementioned alkyl radicals, and also, for example, the nonyl, 1-decyl or 2-decyl radical.

"(C₁-C₄)-Haloalkyl" is to be understood as meaning an alkyl group mentioned under the expression "(C₁-C₄)-alkyl" in which one or more hydrogen atoms are replaced by the same number of identical or different halogen atoms, preferably by chlorine or fluorine, such as the trifluoromethyl, the 1-fluoroethyl, the 2,2,2-trifluoroethyl, the chloromethyl, fluoromethyl, the difluoromethyl and the 1,1,2,2-tetrafluoroethyl group.

"(C₁-C₄)-Alkoxy" is to be understood as meaning an alkoxy group whose hydrocarbon radical has the meaning given under the expression "(C₁-C₄)-alkyl". Alkoxy groups having a larger range of carbon atoms are to be understood accordingly.

The terms "alkenyl" and "alkynyl" with a range of carbon atoms stated as prefix denote a straight-chain or branched hydrocarbon radical having a number of carbon atoms which corresponds to this stated range and which contains at least one multiple bond which can be located in any position of the respective unsaturated radical. "(C₂-C₄)-Alkenyl" accordingly denotes, for example, the vinyl, allyl, 2-methyl-2-propenyl or 2-butenyl group; "(C₂-C₆)-Alkenyl" denotes the abovementioned radicals and also, for example, the pentenyl, 2-methylpentenyl or the hexenyl group. "(C₂-C₄)-Alkynyl" denotes, for example, the ethynyl, propargyl, 2-methyl-2-propynyl or 2-butynyl group. "(C₂-C₆)-Alkynyl" is to be understood as meaning the abovementioned radicals and also, for example, the 2-pentynyl or the 2-hexynyl group, and "(C₂-C₁₀)-alkynyl" is to be understood as meaning the abovementioned radicals and also, for example, the 2-octynyl or the 2-decynyl group.

"(C₃-C₈)-Cycloalkyl" denotes monocyclic alkyl radicals, such as the cyclopropyl, cyclobutyl, cyclopentyl, cyclohexyl, cycloheptyl or cyclooctyl radical, and denotes bicyclic alkyl radicals, such as the norbornyl radical.

The expression "(C₃-C₈)-cycloalkyl-(C₁-C₄)-alkyl" is to be understood as meaning, for example the cyclopropylmethyl, cyclopentylmethyl, cyclohexylmethyl, cyclohexylethyl and cyclohexylbutyl radical, and the expression "(C₁-C₆)-alkyl-(C₃-C₈)-cycloalkyl" is to be understood as meaning, for example, the 1-methylcyclopropyl, 1-methylcyclopentyl, 1-methylcyclohexyl, 3-hexylcyclobutyl and the 4-tert-butylcyclohexyl radical.

"(C₁-C₄)-Alkoxy-(C₁-C₆)-alkyloxy" denotes an alkoxy group as defined above which is substituted by a further alkoxy group, such as, for example, 1-ethoxyethoxy.

"(C₃-C₈)-Cycloalkoxy" or "(C₃-C₈)-cycloalkylthio" is to be understood as meaning one of the abovementioned (C₃-C₈)-cycloalkyl radicals which is attached via an oxygen or sulfur atom.

"(C₃-C₈)-Cycloalkyl-(C₁-C₆)-alkoxy" denotes, for example, the cyclopropylmethoxy, cyclobutylmethoxy, cyclopentylmethoxy, cyclohexylmethoxy, cyclohexylethoxy or the cyclohexylbutoxy group.

The expression "(C₁-C₄)-alkyl-(C₃-C₈)-cycloalkoxy" denotes, for example, the methylcyclopropyloxy, methylcyclobutyloxy or the butylcyclohexyloxy group.

"(C₁-C₆)-Alkylthio" denotes an alkylthio group whose hydrocarbon radical has the meaning given under the expression "(C₁-C₆)-alkyl".

Similarly, "(C₁-C₆)-alkylsulfinyl" denotes, for example, the methyl, ethyl, propyl, isopropyl, butyl, isobutyl, sec-butyl- or tert-butylsulfinyl group and "(C₁-C₆)-alkylsulfonyl" denotes, for example, the methyl, ethyl, propyl, isopropyl, butyl, isobutyl, sec-butyl- or tert-butylsulfonyl group.

"(C₁-C₆)-Alkylamino" denotes a nitrogen atom which is substituted by one or two identical or different alkyl radicals of the above definition.

The expression "(C₁-C₆)-mono- or -dialkylcarbamoyl" denotes a carbamoyl group having one or two hydrocarbon radicals which have the meaning given under the expression "(C₁-C₆)-alkyl" and which, in the case of two hydrocarbon radicals, can be identical or different.

Similarly, "(C₁-C₆)-dihaloalkylcarbamoyl" denotes a carbamoyl group which carries two (C₁-C₆)-haloalkyl radicals according to the above definition or one (C₁-C₆)-haloalkyl radical and one (C₁-C₆)-alkyl radical according to the above definition.

"(C₁-C₆)-Alkanoyl" denotes, for example, the formyl, acetyl, propionyl, butyryl or 2-methylbutyryl group.

The expression "aryl" is to be understood as meaning a carbocyclic, i.e. constructed of carbon atoms, aromatic radical having preferably 6 to 14, in particular 6 to 12, carbon atoms, such as, for example, phenyl, naphthyl or biphenylyl, preferably phenyl. "Aroyl" accordingly denotes an aryl radical as defined above which is attached via a carbonyl group, such as, for example, the benzoyl group.

The expression "heterocyclyl" preferably denotes a cyclic radical which can be completely saturated, partially unsaturated or completely unsaturated and which can be interrupted by at least one or more identical or different atoms selected from the group consisting of nitrogen, sulfur and oxygen, where, however, two oxygen atoms may not be directly adjacent and at least one carbon atom has to be present in the ring, such as, for example, a thiophene, furan, pyrrole, thiazole, oxazole, imidazole, isothiazoe, isoxazole, pyrazole, 1,3,4-oxadiazole, 1,3,4-thiadiazole, 1,3,4-triazole, 1,2,4-oxadiazole, 1,2,4-thiadiazole, 1,2,4-triazole, 1,2,3-triazole, 1,2,3,4-tetrazole, benzo[b]thiophene, benzo[b]furan, indole, benzo[c]thiophene, benzo[c]furan, isoindole, benzoxazole, benzothiazole, benzimidazole, benzisoxazole, benzisothiazole, benzopyrazole, benzothiadiazole, benzotriazole, dibenzofuran, dibenzothiophene, carbazole, pyridine, pyrazine, pyrimidine, pyridazine, 1,3,5-triazine, 1,2,4-triazine, 1,2,4,5-tetrazine, quinoline, isoquinoline, quinoxaline, quinazoline, cinnoline, 1,8-naphthyridine, 1,5-naphthyridine, 1,6-naphthyridine, 1,7-naphthyridine, phthalazine, pyridopyrimidine, purine, pteridine, 4H-quinolizine, piperidine, pyrrolidine, oxazoline, tetrahydrofuran, tetrahydropyran, isoxazolidine or thiazolidine radical. Accordingly, the expression "heteroaromatic" embraces, from among the meanings mentioned above under "heterocyclyl", in each case the completely unsaturated aromatic heterocyclic compounds.

Heterocyclyl particularly preferably denotes a saturated, partially saturated or aromatic ring system having 3 to 6 ring members and 1 to 4 heteroatoms selected from the group consisting of O, S and N, where at least one carbon atom has to be present in the ring.

Very particularly preferably, heterocyclyl denotes a pyridine, pyrimidine, (1,2,4)-oxadiazole, (1,3,4)-oxadiazole, pyrrole, furan, thiophene, oxazole, thiazole, imidazole, pyrazole, isoxazole, 1,2,4-triazole, tetrazole, pyrazine, pyridazine, oxazoline, thiazoline, tetrahydrofuran, tetrahydropyran, morpholine, piperidine, piperazine, pyrroline, pyrrolidine, oxazolidine, thiazolidine, oxirane and oxetane radical.

"Aryl-(C₁-C₄)-alkoxy" denotes an aryl radical which is attached via a (C₁-C₄)-alkoxy group, for example the benzyloxy, phenylethoxy, phenylbutoxy or naphthylmethoxy radical.

"Arylthio" denotes an aryl radical which is attached via a sulfur atom, for example the phenylthio or the 1- or 2-naphthylthio radical. Similarly, "aryloxy" denotes, for example, the phenoxy or 1- or 2-naphthyloxy radical.

"Aryl-(C₁-C₄)-alkylthio" denotes an aryl radical which is attached via an alkylthio radical, for example the benzylthio, naphthylmethylthio or the phenylethylthio radical.

The expression "(C₁-C₆)-trialkylsilyl" denotes a silicon atom which carries three identical or different alkyl radicals according to the above definition. Similarly, "aryl-(C₁-C₆)-dialkylsilyl" denotes a silicon atom which carries one aryl radical and two identical or different alkyl radicals according to the above definition, "diaryl-(C₁-C₆)-alkylsilyl" denotes a silicon atom which carries one alkyl radical and two identical or different aryl radicals according to the above definition and "triarylsilyl" denotes a silicon atom which carries three identical or different aryl radicals according to the above definition.

The compounds of the formula (I) and (II) can be obtained by methods well known in the art and described e.g. in Horben-Weyl, Methoden der Organischen Chemie, G. Thieme Verlag, Stuttgart, Germany.

The compounds of Formulae (I) and (II) where A is N-O-CHR³ and m is zero can be prepared by reacting a ketone of the following Formulae (VII) and (VIII) and with an amine of the formula NH₂O-CHR³-B wherein B has the meanings defined in Formulas (I) and (II) as depicted below:

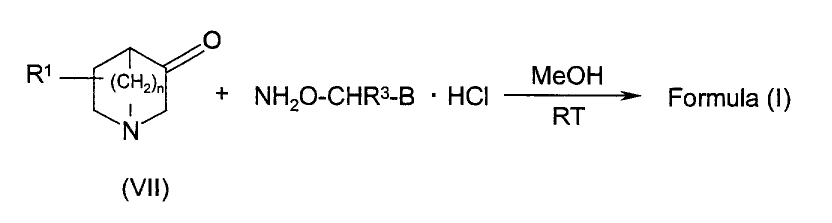

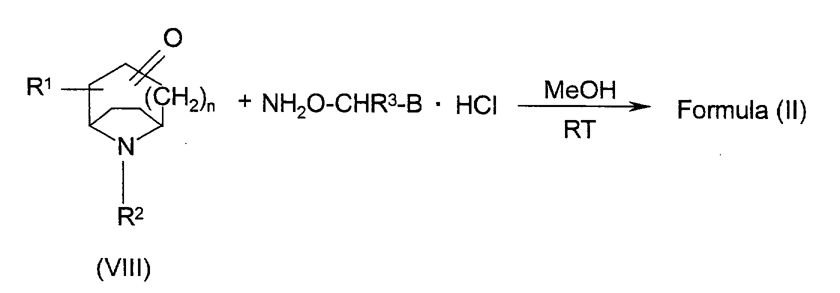

In each of the above depicted reactions, typically methanol is used as the solvent and the reaction is carried out at room temperature or at elevated temperature, such as reflux.

Compounds of formula (I) and (II) where A is N-O-CH₂ and m is zero can be obtained as outlined in scheme 1 for compounds of formula (III):

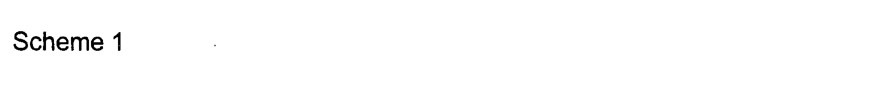

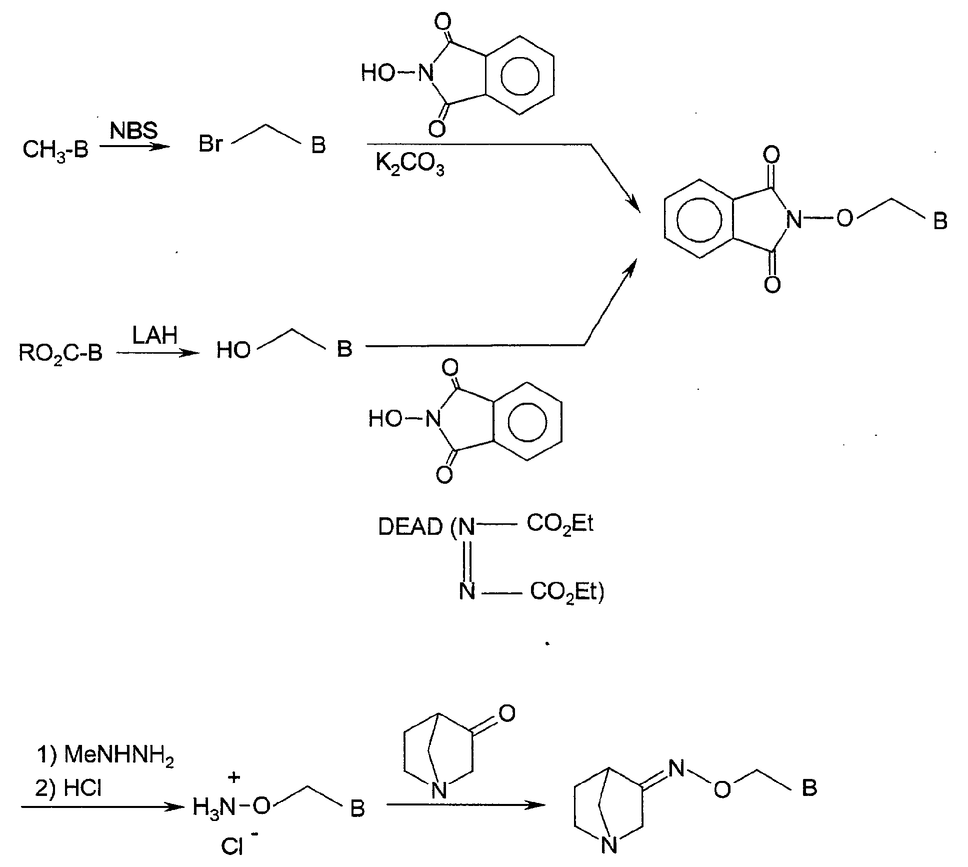

Compounds of formula (I) and (II) where A is =CH and m is zero can be obtained as outlined in scheme 2 for compounds of formula (III):

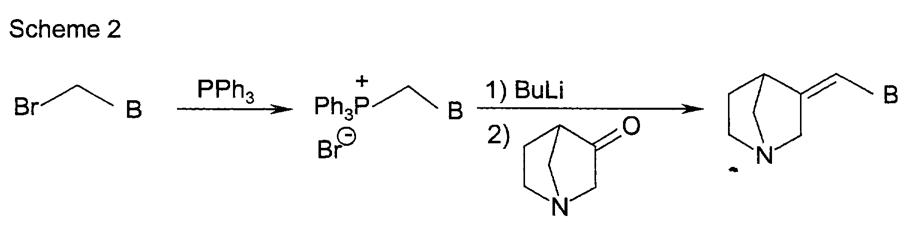

Compounds of formulae (I) and (II) where m is one can be obtained by oxidation of compounds of formulae (I) and (II) where m is zero, typically with a peracid or peroxide in a chlorinated hydrocarbon or aqueous alcohol solvent at room temperature.

The salts can be prepared by contacting the free base form of the compounds of the invention with a sufficient amount of the desired acid to produce the salt in the conventional manner. The free base forms may be regenerated, if desired, by treating the salt form with a base. For example, dilute aqueous solutions of such bases as sodium hydroxide, potassium carbonate, ammonia, and sodium bicarbonate may be utilized for this purpose.

The free base forms of the compounds of this invention differ somewhat from their respective salt forms in such physical properties as melting point and solubility in polar solvents, but the salts are otherwise equivalent to their respective free acid or base forms for the use according to the invention.

The starting materials represented by Formulae (VII), and (VIII) are known in the art or can be prepared by procedures generally known in the art. See, for example, K. Nádor et al., Arzneim. Forsch., 1962, 12, 305 and J.Sauders et al., J. Chem. Soc., Chem. Comm. 1988, 1618 and R.J. Snow and L.J. Street, Tet. Lett. 1989, 30, 5795 and L. J. Street et al., J. M. ed. Chem., 1990, 33, 2690.

Collections of compounds of the formulae (I) and (II) which can be synthesized by the abovementioned schemes may also be prepared in a parallel manner, and this may be effected manually or in a semiautomated or fully automated manner. In this case, it is possible, for example, to automate the procedure of the reaction, work-up or purification of the products or of the intermediates. In total, this is to be understood as meaning a procedure as it is described, for example, by S.H. DeWitt in "Annual Reports in Combinatorial Chemistry and Molecular Diversity: Automated synthesis", Volume 1, Verlag Escom 1997, pages 69 to 77.

Commercially available apparatus as offered by, for example, Stem Corporation, Woodrolfe Road, Tollesbury, Essex, CM9 8SE, England or H+P Labortechnik GmbH, Bruckmannring 28, 85764 Oberschleißheim, Germany, may be used for the parallel procedure of the reaction and work-up. For the parallel purification of compounds of the formulae (I) to (IV), or of intermediates obtained during the preparation, use may be made, inter alia, of chromatography apparatus, for example from ISCO, Inc., 4700 Superior Street, Lincoln, NE 68504, USA.

The apparatus mentioned leads to a modular procedure in which the individual process steps are automated, but manual operations must be performed between the process steps. This can be prevented by employing semi-integrated or fully integrated automation systems where the automation modules in question are operated by, for example, robots. Such automation systems can be obtained, for example, from Zymark Corporation, Zymark Center, Hopkinton, MA 01748, USA.

In addition to what has been described here, compounds of the formulae (I) to (IV) may be prepared in part or fully by solid-phase-supported methods. For this purpose, individual intermediate steps or all intermediate steps of the synthesis or of a synthesis adapted to suit the procedure in question are bound to a synthetic resin. Solid-phase-supported synthesis methods are described extensively in the specialist literature, for example Barry A. Bunin in "The Combinatorial Index", Academic Press, 1998.

The use of solid-phase-supported synthesis methods permits a series of protocols which are known from the literature and which, in tum, can be performed manually or in an automated manner. For example, the "tea-bag method" (Houghten, US 4,631,211; Houghten et al., Proc. Natl. Acad. Sci, 1985, 82, 5131-5135), in which products by IRORI, 11149 North Torrey Pines Road, La Jolla, CA 92037, USA, are employed, may be semiautomated. The automation of solid-phase-supported parallel syntheses is performed successfully, for example, by apparatuses by Argonaut Technologies, Inc., 887 Industrial Road, San Carlos, CA 94070, USA or MultiSynTech GmbH, Wullener Feld 4, 58454 Witten, Germany.

The preparation of the processes described herein yields compounds of the formulae (I) and (II) in the form of substance collections which are termed libraries.

In a further aspect of the invention there is provided the use of an effective amount of a compound of the formula (I) and/or (II) for controlling animal pests.

The compounds of the Formulae (I) and (II) are suitable for controlling animal pests, in particular arthropods, e.g. insects and arachnids, helminths, nematodes, ecto- and endoparasites and mollusks, especially for controlling insects and arachnids which are encountered in agriculture, in livestock breeding, in forests, in the protection of stored goods and materials, and in the hygiene sector. They are active against normally sensitive and resistant species and against all or individual developmental stages. The abovementioned pests include:
from the order of the Acarina, for example, Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp.
From the order of the Isopoda, for example, Oniscus aselus, Armadium vulgare, Porcellio scaber.
From the order of the Diplopoda, for example, Blaniulus guttulatus.
From the order of the Chilopoda, for example, Geophilus carpophagus, Scutigera spp.
From the order of the Symphyla, for example, Scutigerella immaculata.
From the order of the Thysanura, for example, Lepisma saccharina.
From the order of the Collembola, for example, Onychiurus armatus.
From the order of the Orthoptera, for example, Blatta orientalis, Periplanata americana, Leucophaea maderae, Blattella germanica, Acheta domesticus, Gryllotalpa spp., Locusta migratoria migratorioides, Melanoplus differentialis, Schistocerca gregaria.
From the order of the Isoptera, for example, Reticulitermes spp.
From the order of the Anoplura, for example, Phylloera vastatrix, Pemphigus spp., Pediculus humanus corporis, Haematopinus spp., Linognathus spp.
From the order of the Mallophaga, for example, Trichodectes pp., Damalinea spp.
From the order of the Thysanoptera, for example, Hercinothrips femoralis, Thrips tabaci.
From the order of the Heteroptera, for example, Eurygaster spp., Dysdercus intermedius, Piesma quadrata, Cimex lectularius, Rhodnius prolixus, Triatoma spp.
From the order of the Homoptera, for example, Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
From the order of the Lepidoptera, for example, Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

From the order of the Coleoptera, for example, Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylloides chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrynchus assimilis, Hypera postica, Dermestes spp., Trogoderma, Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
From the order of the Hymenoptera, for example, Diprion spp., Hoplocampa spp., Lasius spp., Monomorium pharaonis, Vespa spp.
From the order of the Diptera, for example, Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hypobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
From the order of the Siphonaptera, for example, Xenopsylla cheopsis, Ceratophyllus spp.
From the order of the Arachnida, for example, Scorpio maurus, Latrodectus mactans.
From the class of the helminths, for example, Haemonchus, Trichostrongulus, Ostertagia, Cooperia, Chabertia, Strongyloides, Oesophagostomum, Hyostrongulus, Ancylostoma, Ascaris and Heterakis and also Fasciola.

From the class of the Gastropoda, for example, Deroceras spp., Arion spp., Lymnaea spp., Galba spp., Succinea spp., Biomphalaria spp., Bulinus spp., Oncomelania spp.
From the class of the Bivalva, for example, Dreissena spp.

The plant-parasitic nematodes which can be controlled in accordance with the invention include, for example, the root-parasitic soil-dwelling nematodes such as, for example, those of the genera Meloidogyne (root knot nematodes, such as Meloidogyne incognita, Meloidogyne hapla and Meloidogyne javanica), Heterodera and Globodera (cyst-forming nematodes, such as Globodera rostochiensis, Globodera pallida, Heterodera trifolii) and of the genera Radopholus, such as Radopholus similis, Pratylenchus such as Pratylenchus neglectus, Pratylenchus penetrans and Pratylenchus curvitatus;
Tylenchulus such as Tylenchulus semipenetrans, Tylenchorhynchus, such as Tylenchorhynchus dubius and Tylenchorhynchus claytoni, Rotylenchus such as Rotylenchus robustus, Heliocotylenchus such as Haliocotylenchus multicinctus, Belonoaimus such as Belonoaimus longicaudatus, Longidorus such as Longidorus elongatus, Trichodorus such Trichodorus primitivus and Xiphinema such as Xiphinema index.

Other nematode genera which can be controlled using the compounds according to the invention are Ditylenchus (stem parasites, such as Ditylenchus dipsaci and Ditylenchus destructor), Aphelenchoides (foliar nematodes, such as Aphelenchoides ritzemabosi) and Anguina (seed nematodes, such as Anguina tritici).

The invention also relates to compositions, for example crop protection compositions, preferably insecticidal, acaricidal, ixodicidal, nematicidal, molluskicidal or helminthicidal, especially preferably insecticidal and acaricidal, compositions which comprise one or more compounds of the formulae (I) and/or (II) in addition to suitable formulation auxiliaries.

In general, the compositions according to the invention comprise 1 to 95% by weight of one or more active substances of the Formulae (I) and/or (II).

To prepare the compositions according to the invention, the active substance and the other additives are combined and brought into suitable use form.

They can be formulated in various ways, depending on the biological and/or chemical-physical parameters which prevail. The following are examples of possible formulations:

Wettable powders (WP), emulsifiable concentrates (EC), aqueous solutions (SL), emulsions, sprayable solutions, oil- or water-based dispersions (SC), suspoemulsions (SE), dusts (DP), seed-dressing products, granules in the form of microgranules, spray granules, coated granules and absorption granules, water-dispersible granules (WG), ULV formulations, microcapsules, waxes or baits.

These individual types of formulations are known in principle and are described, for example, in:
Winnacker-Küchler, "Chemical Technology", Volume 7, C. Hanser Verlag Munich, 4th Edition 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

The necessary formulation auxiliaries such as inert materials, surfactants, solvents and other additives, are also known and described, for example, in Watkins, "Handbook of Insecticide Dust Diluents and Carriers", 2nd Ed., Darland Books, Caldwell N.J.; H. v. Olphen, "Introduction to Clay Colloid Chemistry", 2nd Ed., J. Wiley & Sons, N.Y.; Marsden, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", [Surface-active ethylene oxide adducts] Wiss. Verlagsgesell., Stuttgart 1967; Winnacker-Küchler, "Chemische Technologie", Volume 7, C. Hanser Verlag Munich, 4th Edition 1986.

Based on these formulations, it is also possible to prepare combinations with other pesticidally active materials, fertilizers and/or growth regulators, for example in the form of a ready-mix formulation or a tank mix. Wettable powders are preparations which are uniformly dispersible in water which, besides the active substance, also comprise wetters, for example polyoxyethylated alkylphenols, polyoxyethylated fatty alcohols, alkylsulfonates or alkylphenolsulfonates and dispersants, for example sodium lignosulfonate or sodium 2,2'- dinaphthylmethane-6,6'-disulfonate, in addition to a diluent or inert material.

Emulsifiable concentrates are prepared by dissolving the active substance in an organic solvent, for example butanol, cyclohexanone, dimethylformamide, xylene or else higher-boiling aromatics or hydrocarbons, with addition of one or more emulsifiers. As emulsifiers, the following can be used, for example: calcium alkylarylsulfonates such as calcium dodecylbenzenesulfonate, or nonionic emulsifiers such as fatty acid polyglycol esters, alkylaryl polyglycol ethers, fatty alcohol polyglycol ethers, propylene oxide/ethylene oxide condensates, alkyl polyethers, sorbitan fatty acid esters, polyoxyethylene sorbitan fatty acid esters or polyoxyethylene sorbitol esters.

Dusts can be obtained, for example, by grinding the active substance with finely divided solid materials, for example talc or natural clays, such as kaolin, bentonite, pyrophyllite or diatomaceous earth. Granules can be prepared either by atomizing the active substance onto adsorptive, granulated inert material or by applying active substance concentrates onto the surface of carrier materials such as sand or kaolinites, or of granulated inert material, by means of adhesives, for example polyvinyl alcohol or sodium polyacrylate, or else mineral oils. Suitable active substances can also be granulated in the manner which is customary for the preparation of fertilizer granules, if desired as a mixture with fertilizers.

The active substance concentration in wettable powders is, for example, approximately 10 to 90% by weight, the remainder to 100% by weight is composed of customary formulation auxiliaries. In the case of emulsifiable concentrates, the active substance concentration may be approximately 5 to 80% by weight. Formulations in the form of dusts usually comprise 5 to 20% by weight of active substance, sprayable solutions approximately 2 to 20% by weight. In the case of granules, the active substance content depends partly on whether the active compound is in liquid or solid form and on which granulation auxiliaries, fillers and the like are being used.

Besides, the abovementioned active substance formulations comprise, if appropriate, the tackifiers, wetters, dispersants, emulsifiers, penetrants, solvents, fillers or carriers which are conventional in each case.

For use, the concentrates, which are present in commercially available form, are, if desired, diluted in the customary manner, for example in the case of wettable powders, emulsifiable concentrates, dispersions and in some cases also microgranules using water. Preparations in the form of dusts and granules and sprayable solutions are usually not diluted any further with other inert substances prior to use.

The application rate required varies with the external conditions such as, inter alia, temperature and humidity. It may vary within wide limits, for example between 0.0005 and 10.0 kg/ha or more of active substance, but it is preferably between 0.001 and 5 kg/ha.

The active substances according to the invention, in their commercially available formulations and in the use forms prepared from these formulations (see the abovementioned compositions) may be present in mixtures with other active substances such as insecticides, attractants, sterilants, acaricides, nematicides, fungicides, molluskicides, growth regulatory substances or herbicides.

The pesticides include, for example, phosphoric esters, carbamates, carboxylic esters, formamidines, tin compounds and materials produced by microorganisms.

Preferred components in mixtures are:
1. from the group of the phosphorus compounds
   acephate, azamethiphos, azinphos-ethyl, azinphos-methyl, bromophos, bromophos-ethyl, cadusafos (F-67825), chlorethoxyphos, chlorfenvinphos, chlormephos, chlorpyrifos, chlorpyrifos-methyl, demeton, demeton-S-methyl, demeton-S-methyl sulfone, dialifos, diazinon, dichlorvos, dicrotophos, dimethoate, disulfoton, EPN, ethion, ethoprophos, etrimfos, famphur, fenamiphos, fenitriothion, fensulfothion, fenthion, fonofos, formothion, fosthiazate (ASC-66824), heptenophos, isazophos, isothioate, isoxathion, malathion, methacrifos, methamidophos, methidathion, salithion, mevinphos, monocrotophos, naled, omethoate, oxydemeton-methyl, parathion, parathion-methyl, phenthoate, phorate, phosalone, phosfolan, phosphocarb (BAS-301), phosmet, phosphamidon, phoxim, pirimiphos, pirimiphos-ethyl, pirimiphos-methyl, profenofos, propaphos, proetamphos, prothiofos, pyraclofos, pyridapenthion, quinalphos, sulprofos, temephos, terbufos, tebupirimfos, tetrachlorvinphos, thiometon, triazophos, trichlorphon, vamidothion;
2. from the group of the carbamates
   alanycarb (OK-135), aldicarb, 2-sec-butylphenyl methylcarbamate (BPMC), carbaryl, carbofuran, carbosulfan, cloethocarb, benfuracarb, ethiofencarb, furathiocarb, HCN-801, isoprocarb, methomyl, 5-methyl-m-cumenylbutyryl (methyl)carbamate, oxamyl, pirimicarb, propoxur, thiodicarb, thiofanox, 1-methylthio(ethylideneamino)-N-methyl-N-(morpholinothio)carbamate (UC 51717), triazamate;
3. from the group of the carboxylic esters
   acrinathrin, allethrin, alphametrin, 5-benzyl-3-furylmethyl (E)- (1R)-cis-2,2-dimethyl-3-(2-oxothiolan-3-ylidenemethyl)cyclopropanecarboxylate, beta-cyfluthrin, beta-cypermethrin, bioallethrin, bioallethrin ((S)-cyclopentylisomer), bioresmethrin, bifenthrin, (RS)-1-cyano-1-(6-phenoxy-2-pyridyl)methyl (1RS)-trans-3-(4-tert-butylphenyl)-2,2-dimethylcyclopropanecarboxylate (NCI 85193), cycloprothrin, cyfluthrin, cyhalothrin, cythithrin, cypermethrin, cyphenothrin, deltamethrin, empenthrin, esfenvalerate, fenfluthrin, fenpropathrin, fenvalerate, flucythrinate, flumethrin, fluvalinate (D isomer), imiprothrin (S-41311), lambda-cyhalothrin, permethrin, phenothrin ((R) isomer), prallethrin, pyrethrins (natural products), resmethrin, tefluthrin, tetramethrin, theta-cypermethrin (TD-2344), tralomethrin, transfluthrin, zeta-cypermethrin (F-56701);
4. from the group of the amidines
   amitraz, chlordimeform;
5. from the group of the tin compounds
   cyhexatin, fenbutatin oxide;
6. others
   abamectin, ABG-9008, acetamiprid, anagrapha falcitera, AKD-1022, AKD-3059, ANS-118, bacillus thuringiensis, beauveria bassianea, bensultap, bifenazate (D-2341), binapacryl, BJL-932, bromopropylate, BTG-504, BTG-505, buprofezin, camphechlor, cartap, chlorobenzilate, chlorfenapyr, chlorfluazuron, 2-(4-chlorophenyl)-4,5-diphenylthiophene (UBI-T 930), chlorfentezine, chromafenozide (ANS-118), CG-216, CG-217, CG-234, A-184699, 2-naphthylmethyl cyclopropanecarboxylate (Ro12-0470), cyromazin, diacloden (thiamethoxam), diafenthiuron, ethyl 2-chloro-N-(3,5-dichloro-4-(1,1,2,3,3,3-hexafluoro-1-propyloxy)phenyl)carbamoyl)-2-carboximidate, dicofol, diflubenzuron, N-(2,3-dihydro-3-methyl-1,3-thiazol-2-ylidene)-2,4-xylidine, dinobuton, dinocap, diofenolan, DPX-062, emamectin benzoate (MK-244), endosulfan, ethiprole (sulfethiprole), ethofenprox, etoxazole (YI-5301), fenazaquin, fenoxycarb, fipronil, fluazuron, flumite (flufenzine, SZI-121), 2-fluoro-5-(4-(4-ethoxyphenyl)-4-methyl-1-pentyl)diphenyl ether (MTI 800), granulosis and nuclear polyhedrosis viruses, fenpyroximate, fenthiocarb, flubenzimine, flucycloxuron, flufenoxuron, flufenprox (ICI-A5683), fluproxyfen, gamma-HCH, halofenozide (RH-0345), halofenprox (MTI-732), hexaflumuron (DE_473), hexythiazox, HOI-9004, hydramethylnon (AC 217300), lufenuron, imidacloprid, indoxacarb (DPX-MP062), kanemite (AKD-2023), M-020, MTI-446, ivermectin, M-020, methoxyfenozide (intrepid, RH-2485), milbemectin, NC-196, neemgard, nitenpyram (TI-304), 2-nitromethyl-4,5-dihydro-6H-thiazine (DS 52618), 2-nitromethyl-3,4-dihydrothiazole (SD 35651), 2-nitromethylene-1,2-thiazinan-3-ylcarbamaldehyde (WL 108477), pyriproxyfen (S-71639), NC-196, NC-1111, NNI-9768, Novaluron (MCW-275), OK-9701, OK-9601, OK-9602, propargite, pymethrozine, pyridaben, pyrimidifen (SU-8801), RH-0345, RH-2485, RYI-210, S-1283, S-1833, SB7242, SI-8601, silafluofen, silomadine (CG-177), spinosad, SU-9118, tebufenozide, tebufenpyrad (MK-239), teflubenzuron, tetradifon, tetrasul, thiacloprid, thiocyclam, TI-435, tolfenpyrad (OMI-88), triazamate (RH-7988), triflumuron, verbutin, vertalec (mykotal), YI-5301.

The active substance content of the use forms prepared from the commercially available formulations may range from 0.00000001 up to 95% by weight of active substance, preferably between 0.00001 and 1% by weight. Application is effected in a customary manner adapted to suit the use forms.

The invention also relates to a method of controlling animal pests, preferably harmful insects, Acarina, helminths, mollusks and/or nematodes in which an effective amount of one or more compounds of formulae (I) and (II) or of a composition comprising of one or more compounds of formulae (I) and (II) is applied to the animal pests or to the plants, areas or substrates infested with them.

The compounds of formulae (I) and (II) are also suitable for use in the veterinary medicine sector, preferably for controlling endoparasites and ectoparasites, and in the field of animal keeping. In a further aspect of the invention there is provided the use of a compound of the formula (I) and (II) in the preparation of a veterinary medicine, preferably for treating ecto- and endoparasites.

The active substances according to the invention may be applied in the known manner, such as by oral administration in the form of, for example, tablets, capsules, drinks or granules, by dermal application in the form of, for example, dipping, spraying, pouring on and spotting on, and dusting, and by parenteral administration in the form of, for example, an injection.

In addition, the compounds of formula (I) and (II) are also suitable for use in technology, for example as wood preservative, as preservative in paints, in cooling lubricants for metalworking, or as preservative in drilling and cutting oils.

Accordingly, the compounds of formulae (I) and (II) can also be employed particularly advantageously in livestock keeping (for example cattle, sheep, pigs and poultry such as chickens, geese and the like). In a preferred embodiment of the invention, the compounds, if appropriate in suitable formulations (cf. above), are administered orally to the animals, if appropriate together with the drinking water or feed. Since excretion in the feces is highly efficient, the development of insects in the animals' feces can be prevented very easily in this manner. The dosages and formulations which are suitable in each case depend, in particular, on the species and the developmental stage of the productive livestock and also on the risk of infestation and can be determined readily and established by customary methods. For example, the compounds can be employed in cattle at dosages of 0.01 to 1 mg/kg bodyweight.

In addition the compounds or compositions according to the invention can be applied in combination with fungicides known from the literature.

Such fungicides are described e.g. in C.D.S. Tomlin, S.B. Walker, The Pesticide Manual, 11^{th} ed., British Crop Protection Council, Farnham 1997.

The invention also relates to seed, treated or coated with an effective amount of one or more compounds of formulae (I) and (II) or of a composition according to the invention.

The compounds of the formulae (I) and (II) can also be employed for controlling harmful organisms in crops of known genetically engineered plants or genetically engineered plants yet to be developed. As a rule, the transgenic plants are distinguished by especially advantageous properties, for example by resistances to particular crop protection agents, resistances to plant diseases or pathogens of plant diseases, such as particular insects or microorganisms such as fungi, bacteria or viruses. Other particular properties concern, for example, the harvested material with regard to quantity, quality, storage properties, composition and specific constituents.

The use in economically important transgenic crops of useful plants and omamentals is preferred, for example of cereals such as wheat, barley, rye, oats, millet, rice, cassava and maize or else crops of sugar beet, cotton, soya, oilseed rape, potatoes, tomatoes, peas and other types of vegetables.

When used in transgenic crops, in particular those which have resistances to insects, effects are frequently observed, in addition to the effects against harmful organisms to be observed in other crops, which are specific for application in the transgenic crop in question, for example an altered or specifically widened spectrum of pests which can be controlled, or altered application rates which may be employed for application.

The invention therefore also relates to the use of compounds of the formulae (I) and (II) for controlling harmful organisms in transgenic crop plants.

The use according to the invention of compounds of the formula (I) or formula (II) or of compositions comprising them, for example as insecticide, acaricide, molluskicide, nematicide or helminthicide, also includes the case where the compound of the formula (I) or formula (II) or its salt is formed from a precursor only after application, for example in the insect, in a plant or in the soil.

The invention will now be illustrated, but in no way limited, by the following Examples.

### Example 1

### 1-Azabicyclo [2.2.1]hept-3-one O-(5-ethoxy-1,2,3-thiadiazol-4-ylmethyl)oxime hydrochloride

A solution of 1-azabicyclo [2.2.1]hept-3-one (0.04 g, 0.18 mmol) and 4-ammonioxymethyl-5-ethoxy-1,2,3-thiadiazole hydrochloride (0.04 g, 0.18 mmol) in methanol (2.5 ml) was heated at 60°C for 6 hrs. The reaction mixture was concentrated in vacuo to give the title compound as a mixture of isomers; m/z (Cl) 269 (M+1).

### Example 2

### 1-Azabicyclo [2.2.1]hept-3-one O-(5-n-propylmercapto-1,2,3-thiadiazol-4-ylmethyl)oxime hydrochloride

A solution of 1-azabicyclo [2.2.1]hept-3-one (0.06 g, 0.27 mmol) and 4-ammoniooxymethyl-5-n-propylmercapto-1,2,3.thiadiazole hydrochloride (0.07 g, 0.27 mmol) in methanol was heated at 60°C for 6 hrs. The reaction mixture was concentrated in vacuo to give the title compound as a mixture of isomers; m/z (Cl) 299 (M+1).

### Example 3

### 1-Azabicyclo[2.2.1]hept-3-one O-(5-chloro-1,2,3-thiadiazolylmethyl)oxime N-oxide

A solution of 1-azabicyclo[2.2.1]hept-3-one O-(5-chloro-1,2,3-thiadiazolylmethyl) oxime (0.14 g; 0.54 mmol) and 3-chloroperbenzoic acid (0.13 g of 70% material, 0.54 mmol) in dichloromethane (15 ml) was stirred overnight at ambient temperature. The reaction mixture was concentrated in vacuo and purified by column chromatography to give the title compound as a mixture of isomers; m/z (positive ion electrospray) 275 (M+1).

### Example 4

### 1-Azabicyclo[2.2.1]hept-3-one O-(5-i-butylmercapto-1,2,3-thiadiazol-4-ylmethyl)oxime

A solution of 1-azabicyclo[2.2.1]hept-3-one O-(5-chloro-1,2,3-thiadiazolylmethyl)oxime (0.12 g, 0.46 mmol), i-butylmercaptan (0.18 ml, 0.46 mmol) and sodium ethoxide (0.18 ml of a 20 % ethanolic solution, 0.46 mmol) in THF (3 ml) was heated at 40°C for 4.5 hrs. The mixture was then evaporated in vacuo and CH₂Cl₂ (3 ml) and water (0.1 ml) were added. The mixture was dried (MgSO₄), filtered and re-evaporated to yield the title compound as a mixture of isomers; m/z (CI) 313 (M+1).

### Example 5

### 1-Azabicyclo[2.2.1]hept-3-one O-(5-carboethoxymethylmercapto-1,2,3-thiadiazol-4-ylmethyl)oxime

A solution of 1-azabicyclo[2.2.1]hept-3-one O-(5-chloro-1,2,3-thiadiazolylmethyl)oxime (0.24 g, 0.92 mmol), mercaptoacetic acid ethyl ester (0.12 g, 1.0 mmol), sodium ethoxide (0.42 ml of a 20 % ethanolic solution, 1.1 mmol) and potassium iodide (20 mg) in absolute ethanol (5 ml) was heated at 90°C for 13 hrs. The mixture was then evaporated in vacuo, redissolved in CH₂Cl₂, washed with water then saturated NaHCO₃, dried (MgSO₄) and evaporated. Column chromatography on silica gel yielded the title compound as a mixture of isomers; m/z (CI) 343 (M+1).

### Example 6

### 1-Azabicyclo[2.2.1]hept-3-one O-(5-cyano-1,2,3-thiadiazol-4-ylmethyl)oxime

A mixture of 1-azabicyclo [2.2.1]hept-3-one O-(5-chloro-1,2,3-thiadiazolylmethyl)oxime (0.13 g, 0.49 mmol), potassium cyanide (0.05 g, 0.73 mmol) and sodium methanesulfinate (0.02 g, 0.16 mmol) in DMF (2 ml) was heated at 80°C for 1 hr. The mixture was then poured into cold water, extracted twice with EtOAc, dried (MgSO₄) and evaporated. Purification using HPLC yielded the title compound as a mixture of isomers; m/z (CI) 250 (M+1).

### Example 7

### 1-Azabicyclo[2.2.1]hept-3-one O-(4-methyl-1,2,3-thiadiazol-5-ylmethyl)oxime hydrochloride

A solution of 1-azabicyclo[2.2.1]hept-3-one (0.05 g, 0.32 mmol) and 5-ammonioxymethyl-4-methyl-1,2,3-thiadiazole hydrochloride (0.06 g, 0.32 mmol) in methanol (3 ml) was heated at 60°C for 5 hrs. The reaction mixture was concentrated in vacuo to give the title compound as a mixture of isomers; m/z (CI) 239 (M+1).

### Example 8

### 1-Azabicyclo[2.2.2]octan-3-one O-(5-ethoxy-1,2,3-thiadiazol-4-ylmethyl)oxime

A solution of 1-azabicyclo[2.2.2]octan-3-one O-(5-chloro-1,2,3-thiadiazolyl-4-ylmethyl)oxime (0.1 g, 0.37 mmol) and sodium ethoxide (0.16 ml of a 20 % ethanolic solution, 0.4 mmol) in absolute ethanol (5 ml) was heated at reflux for 3 hrs. The mixture was evaporated in vacuo, redissolved in CH₂Cl₂, washed with water, dried (MgSO₄) and evaporated to yield the title compound as a mixture of isomers; m/z (CI) 283 (M+1).

### Example 9

### 1-Azabicyclo[2.2.2]octan-3-one O-(5-n-propylmercapto-1,2,3-thiad iazol-4-ylmethyl)oxime

A solution of 1-azabicyclo[2.2.2]octan-3-one (0.11 g, 0.42 mmol), 1-propanthiol (0.03 g, 0.42 mmol) and sodium ethoxide (0.16 ml of a 20 % ethanolic solution, 0.42 mmol) in dry THF (5 ml) was heated at reflux for 3 hrs. The mixture was evaporated in vacuo, redissolved in CH₂Cl₂, washed with water, dried (MgSO₄) and evaporated to yield the title compound as a mixture of isomers; m/z (Cl) 313 (M+1).

### Example 10

### 8-Methyl-8-azabicyclo[3.2.1]octan-3-one O-(4-methyl-1,2,3-thiadiazol-5-ylmethyl)oxime hydrochloride

A solution of tropinone (0.05 g, 0.36 mmol) and 5-ammonioxymethyl-4-methyl-1,2,3-thiadiazole hydrochloride (0.07 g, 0.36 mmol) in methanol (3 ml) was heated at 60°C for 5 hrs. The reaction mixture was concentrated in vacuo to give the title compound as a mixture of isomers; m/z (Cl) 267.

### B. Formulation examples

a) A dust is obtained by mixing 10 parts by weight of active substance and 90 parts by weight of talc as inert material and comminuting the mixture in a hammer mill.
b) A wettable powder which is readily dispersible in water is obtained by mixing 25 parts by weight of active substance, 65 parts by weight of kaolin-containing quartz as inert material, 10 parts by weight of potassium lignosulfonate and 1 part by weight of sodium oleoylmethyltaurinate as wetter and dispersant and grinding the mixture in a pinned-disk mill.
c) A dispersion concentrate which is readily dispersible in water is prepared by mixing 40 parts by weight of active substance with 7 parts by weight of a sulfosuccinic monoester, 2 parts by weight of a sodium lignosulfonate and 51 parts by weight of water and grinding the mixture in a ball mill to a fineness of below 5 microns.
d) An emulsifiable concentrate can be prepared from 15 parts by weight of active substance, 75 parts by weight of cyclohexanone as solvent and 10 parts by weight of oxyethylated nonylphenol (10 EO) as emulsifier.
e) Granules can be prepared from 2 to 15 parts by weight of active substance and an inert granule carrier material such as attapulgite, pumice granules and/or quartz sand. It is expedient to use a suspension of the wettable powder of Example b) with a solids content of 30%, which is sprayed onto the surface of attapulgite granules, and these are dried and mixed intimately. The wettable powder amounts to approx. 5% by weight and the inert carrier material to approx. 95% by weight of the finished granules.

### C. Biological examples

### Example 1 Effect on the spider mite Tetranychus urticae

Bean plants (Phaseolus vulgaris) which were severely infested with a complete population of spider mites (Tetranychus urticae) were dipped into an aqueous solution of the formulated preparation to be tested. After 6 days storage in a controlled-environment cabinet at approx. 25°C, the mortality of all mite stages was determined. At a concentration of 500 ppm (based on the active substance content) a 50 to 100% mortality was caused by the preparations described herein.

### Example 2 Effect on the larval stage of Spodoptera exigua

An aqueous solution (200 µl) of the formulated preparation to be tested was poured into a small beaker containing five L-2 stage Spodoptera exigua larvae. The beaker was then emptied into a petri dish whose bottom was covered with filter paper and which contained approx. 5 ml of nutrient medium. A further 200 µl of the preparation solution was poured over the nutrient medium. After 6 days storage at approx. 25°C, the mortality among the larvae was determined. At a concentration of 500 ppm (based on the active substance content) a 50 to 100 % mortality was caused by the preparations described herein.

### Example 3 Effect on larvae of Nilaparvata lugens

The leaves of 12 rice plants having a stem length of 8 cm are dipped for 5 seconds into an aqueous solution of the formulated preparation to be examined. After the solution has dripped off, the rice plants are placed in a Petri dish and populated with approximately 20 larvae (L3 stage) of the rice leaf hopper species Nilaparvata lugens. After 6 days' storage at approx. 25°C, the mortality among the leaf hopper larvae was determined. At a concentration of 500 ppm (based on the active substance content) a 50 to 100% mortality was caused by the preparations described herein.

### Example 4 Systemic Effect on Aphis fabae

Germinated broad bean seeds (Vicia faba) with radicles are transferred into brown glass bottles filled with tap water. Four milliliters of an aqueous solution of the formulated preparation to be examined are pipetted into the brown glass bottle. The broad bean is subsequently heavily populated with approximately 100 black bean aphids (Aphis fabae). After 6 days' storage at approx. 25°C, the root-systemic activity of the preparation on the aphids is determined. At a concentration of 100 ppm (based on the active substance content) a 50 to 100% mortality was caused by the preparations described herein.

## Claims

1. Azabicyclo compounds of the formulae (I) and (II) or agrochemically acceptable salts thereof 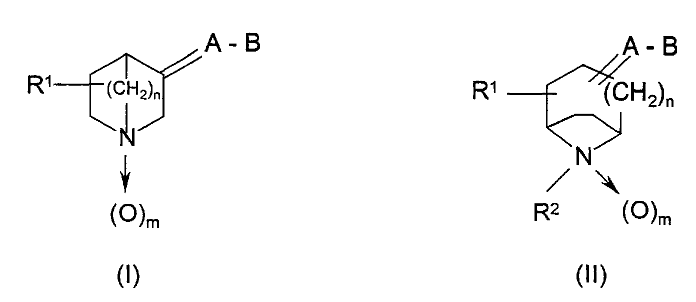 wherein
m is zero or one;
n is one or two;
R¹ is hydrogen, a straight or branched lower alkyl group having from 1 to 6 carbon atoms, (C₃-C₆)-cycloalkyl, hydroxy, a straight or branched lower alkoxy group having from 1 to 4 carbon atoms, an acyloxy group, wherein the acyl moiety has from 2 to 5 carbon atoms;
R² is hydrogen or a straight or branched lower alkyl group having from 1 to 4 carbon atoms, which is optionally substituted by one or more fluorine atoms;
A is N-O-CHR³- or CR⁴;
R³ is H, (C₁-C₄)-alkyl, (C₂-C₄)-alkenyl, (C₂-C₄)-alkynyl, (C₁-C₆)-cycloalkyl;
R⁴ is H, halogen, CN, (C₁-C₄)-alkyl, (C₂-C₄)-alkenyl, (C₂-C₄)-alkynyl, (C₁-C₆)-cycloalkyl, -O-(C₁-C₄)-alkyl, -O-(C₂-C₄)-alkenyl, -O-(C₂-C₄)-alkynyl, -O-(C₁-C₆)-cycloalkyl, -S-(C₁-C₄)-alkyl, -S-(C₂-C₄)-alkenyl, -S-(C₂-C₄)-alkynyl, -S-(C₁-C₆)-cycloalkyl, -NH-(C₁-C₄)-alkyl, -NH-(C₂-C₄)-alkenyl, -NH-(C₂-C₄)-alkynyl, -NH-(C₁-C₆)-cycloalkyl, N((C₁-C₄)-alkyl)₂, N((C₁-C₆)-cycloalkyl)₂, morpholinyl, piperidyl, pyrrolidinyl;
B is 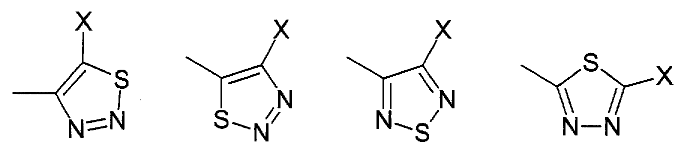 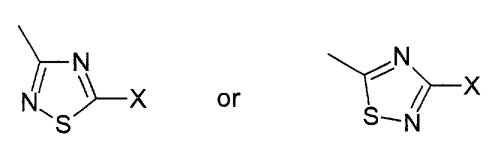
X is H or a substituent.

2. The compound as claimed in claim 1, wherein A is N-O-CHR³.

3. The compound as claimed in claim 1, wherein A is CHR⁴;

4. The compound as claimed in one or more of claims 1 to 3 in which B is

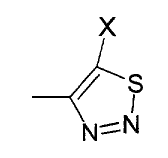

5. A crop protection composition which comprises at least one compound according to formulae (I) and/or (II) in any of claims 1 to 4 and at least one formulation auxiliary.

6. An insecticidal, acaricidal, molluskicidal or nematicidal composition as claimed in claim 5 which comprises an effective amount of at least one compound of formulae (I) and/or (II) according to any of claims 1 to 4 together with additives or auxiliaries conventionally used for this application.

7. A crop protection composition which comprises a insecticidally, acaricidally, molluskicidally or nematicidally effective amount of at least one compound of formulae (I) and/or (III) according to any of claims 1 to 4 and at least one further active substance together with auxiliaries and additives conventionally used for this application.

8. A composition for use in the protection of timber or as preservative in sealants, in paints, in cooling lubricants for metalworking or in drilling and cutting oils, comprising an effective amount of at least one compound of formulae (I) and/or (II) according to any of claims 1 to 4 together with auxiliaries and additives conventionally used for these applications.

9. A compound according to formula (I) and/or (II) in any of claims 1 to 4 or a composition as claimed in claim 6 for use in the preparation of a veterinary medicament for treating ecto- and/or endoparasites.

10. A process for the preparation of a composition as claimed in any of claims 5 to 8, which comprises combining the active substance and the other additives and bringing the mixture into a suitable use form.

11. The use of a compound according to any of claims 1 to 4 or of a composition as claimed in any of claims 5, 6 and 9 as timber preservative or as preservative in sealants, in paints, in cooling lubricants for metalworking or in drilling and cutting oils.

12. A method of controlling harmful insects, acarina, mollusks and nematodes, in which an effective amount of a compound according to formulae (I) and/or (II) in any of claims 1 to 4 or of a composition as claimed in any of claims 5, 6 or 7 is applied to these or to the plants, areas or substrates infested with them.

13. Seed, treated or coated with an effective amount of a compound according to any of claims 1 to 4 or of a composition as claimed in any of claims 5, 6 or 7.
